(19) Europäisches Patentamt

European Patent Office

Office européen des brevets

(11) Numéro de publication: **0 321 350 B1**

(12) **FASCICULE DE BREVET EUROPEEN**

(45) Date de publication de fascicule du brevet: **26.08.92**   (51) Int. Cl.⁵: **G01N 25/48**, G01N 25/02

(21) Numéro de dépôt: **88403204.6**

(22) Date de dépôt: **16.12.88**

(54) **Dispositif de détection d'un phénomène thermique intervenant dans un produit.**

(30) Priorité: **17.12.87 FR 8717626**

(43) Date de publication de la demande:
**21.06.89 Bulletin  89/25**

(45) Mention de la délivrance du brevet:
**26.08.92 Bulletin  92/35**

(84) Etats contractants désignés:
**DE GB IT**

(56) Documents cités:
**DE-C- 1 241 149**
**FR-A- 2 174 328**
**US-A- 3 491 581**
**US-A- 3 552 207**
**US-A- 3 834 873**

**JOURNAL OF PHYSICS E vol. 8, no. 5, mai 1975, pp 377, 378, Bristol, GB, M.B. SMITH et al.: "A scanning calorimeter for thermal analysis of biological materials"**

(73) Titulaire: **INSTITUT FRANCAIS DU PETROLE**
**4, Avenue de Bois Préau**
**F-92506 Rueil-Malmaison Cédex(FR)**

(72) Inventeur: **Claudy, Pierre**
**32, domaine de la Côte**
**F-69530 Brignais(FR)**
Inventeur: **Letoffe, Jean-Marie**
**10, Rue de la Liberté**
**F-69150 Decines Charpieu(FR)**
Inventeur: **Commercon, Jean-Claude**
**4, Chemin du Panorama**
**F-69300 Caluire et Cuire(FR)**

EP 0 321 350 B1

## Description

La présente invention concerne un dispositif permettant d'effectuer la détection et/ou la mesure d'un phénomène thermique intervenant dans un produit qui est à l'état liquide à une température comprise dans la gamme des températures balayées par ce dispositif.

Le dispositif selon la présente invention permet d'obtenir rapidement et précisément les informations recherchées. Par ailleurs, il permet une automatisation aisée de l'acquisition des détections du début d'un effet thermique tel, par exemple, la température d'apparition d'un changement de phase et/ou des mesures plus complètes.

Le dispositif selon la présente invention est particulièrement bien adapté pour la détermination de la température de cristallisation commençante des paraffines dans les carburants diesel.

Les carburants pour moteur diesel ont une importance économique grandissante. On peut grossièrement les assimiler, du point de vue de leur constitution, à une solution de n-paraffines dans une matrice hydrocarbonée complexe. En raison de leur nature, ils posent des problèmes particuliers lorsque leur température d'utilisation est abaissée. Des cristaux de paraffines apparaissent, ce qui entraîne des difficultés, voire l'impossibilité de fonctionnement des moteurs.

Les carburants doivent donc répondre à des impératifs qui sont, en France, établis par la Direction des Carburants. Trois températures sont définies :
- PT : Point de Trouble, ou température d'apparition des premiers cristaux de paraffines.
- TLF : Température Limite de Filtrabilité, à laquelle les cristaux de paraffines obstruent les mailles (45 $\mu$m) du filtre de carburant.
- PE : Point d'Ecoulement, soit la température à laquelle un réseau tridimensionnel de paraffines est créé et le carburant ne peut plus s'écouler.

Des normes américaines (A. S. T. M. ) ou européennes (E. N. ) définissent les dispositifs qui permettent la détermination de ces trois températures. En ce qui concerne le point de trouble, il s'agit de la norme ASTM D-9766. L'appareillage utilisé est très rustique, puisque c'est par observation visuelle directe d'un produit refroidi qu'est détecté le point d'apparition des premiers cristaux de paraffines. La reproductibilité admise est de 4° C.

Des appareils reposant sur le principe de diffusion de la lumière ont été réalisés et sont commercialisés actuellement (par exemple par les sociétés Normandie Labo, ou Malvern, ou Total).

Une autre méthode paraît tout aussi simple : lorsque les premiers cristaux de paraffines se forment au cours d'un refroidissemnt, c'est à dire lorsque, dans le diagramme à n-constituants, on franchit le liquidus, il y a dégagement de chaleur. Ceci est détectable par calorimétrie à balayage (A. C. D. ). Il a été établi qu'il y avait une relation linéaire entre la mesure du point de trouble suivant la norme ASTM et la mesure A. C. D. . De même, la TLF et le PE peuvent être retrouvés par A. C. D. avec cependant des produits non dopés. La reproductibilité des mesures est meilleure que 0,1° C, ce qui est bien meilleur que la norme ASTM.

Dans certains cas la norme conduit à des résultats erronés. En effet, pour obtenir le meilleur rendement d'un brut en produits nobles, les raffineurs utilisent de plus en plus le cracking des fractions lourdes qui conduisent à des huiles claires (L. C. O. ) mélangées ensuite aux fractions distillées. Sur ces carburants, lors d'un refroidissement, on peut observer une démixion (passage de un à deux liquides) interprétée visuellemnt comme étant le point de trouble. Cette erreur n'affecte pas l'A. C. D. , qui observe toujours le début de cristallisation des paraffines.

Le document GB-2 176 011 A décrit un dispositif permettant de connaître les transformations de phase d'un élément, la transformation étant ellemême utilisée pour réguler la température du four. Ce dispositif présente l'inconvénient de travailler sur un échantillon de taille déterminée, qui n'est donc pas représentatif de l'ensemble et nécessite des interventions plus nombreuses lors des opérations de changement d'échantillons à analyser.

Le dispositif selon l'invention notamment dans l'application indiquée précédemment, permet d'atteindre les objectifs suivants :
- Commodité d'emploi :
    Le système calorimétrique peut être utilisé par des personnes peu averties, il importe alors que l'appareil soit autonome. En termes clairs, il ne doit demander aucune opération, en dehors de la mise en marche et de l'approvisionnement en échantillons.
    Le dépouillement des résultats peut être effectué par un programme conçu en fonction des produits à étudier, pour s'affranchir de l'erreur liée à l'observateur, ou à son manque d'expérience.

D'une manière plus générale la présente invention concerne un dispositif de détection et/ou de mesures d'un phénomène thermique intervenant dans un liquide. Ce dispositif comporte une enceinte comportant une tête calorimétrique contenant au moins une cellule et des moyens de mesure d'une grandeur liée à la température de la cellule ou aux échanges de flux de chaleur avec cette cellule, et au moins une cellule de référence, ainsi que des moyens de remplissage de ladite cellule de mesure et de ladite cellule de référence avec ledit liquide et des moyens de vidange de ladite cellule dudit liquide que des moyens de remplissage et de vidange de ladite cellule avec ledit liquide.

Le dispositif selon l'invention comporte en outre des moyens de contrôle de la température régnant dans l'enceinte. Ces moyens qui pourront être qualifiés de moyens de balayage en température, sont adaptés à faire varier la température de l'enceinte où se trouve la cellule de mesure sur intervalle de température prédéterminé.

Cet intervalle de température étant sensé contenir la température pour laquelle le liquide traité devrait être le siège d'un phénomène thermique.

Le dispositif selon l'invention pourra comporter une cellule supplémentaire.

Le dispositif selon l'invention pourra comporter au moins deux cellules de mesure.

Par ailleurs, le dispositif selon l'invention pourra comporter au moins une thermopile placée entre la cellule de mesure et la cellule de référence ou tout autre moyen de mesure de l'échange du flux avec cette cellule tels des fluxmètres.

Les cellules selon la présente invention pourront être placées sur un socle réalisé en un matériau ayant de bonnes caractéristiques de conductibilité thermique.

Ce socle pourra comporter au moins une portion du circuit d'alimentation de ladite cellule en liquide.

Par ailleurs ce socle poura être en contact avec les moyens de transfert thermique.

La présente invention sera mieux comprise et ses avantages apparaîtront plus clairement à la description qui suit d'un exemple particulier nullement limitatif, illustré par les figures, ci-annexées parmis lesquelles :

- la figure 1 illustre dans son ensemble un dispositif selon la présente invention,
- la figure 2 représente en perspective une cellule de mesure,
- la figure 3 montre de manière plus détaillée l'alimentation des cellules,
- la figure 4 montre une vue en perspective de deux cellules sur leur plaque support, ainsi que les canalisations d'alimentation de chacune des cellules et des sondes de mesure,
- la figure 5 représente une tête calorimétrique comportant un écran intermédiaire compensateur,
- la figure 6 illustre un mode de réalisation à trois cellules, et
- la figure 7 montre le schéma de dispositif selon l'invention et de moyens d'automatisation.

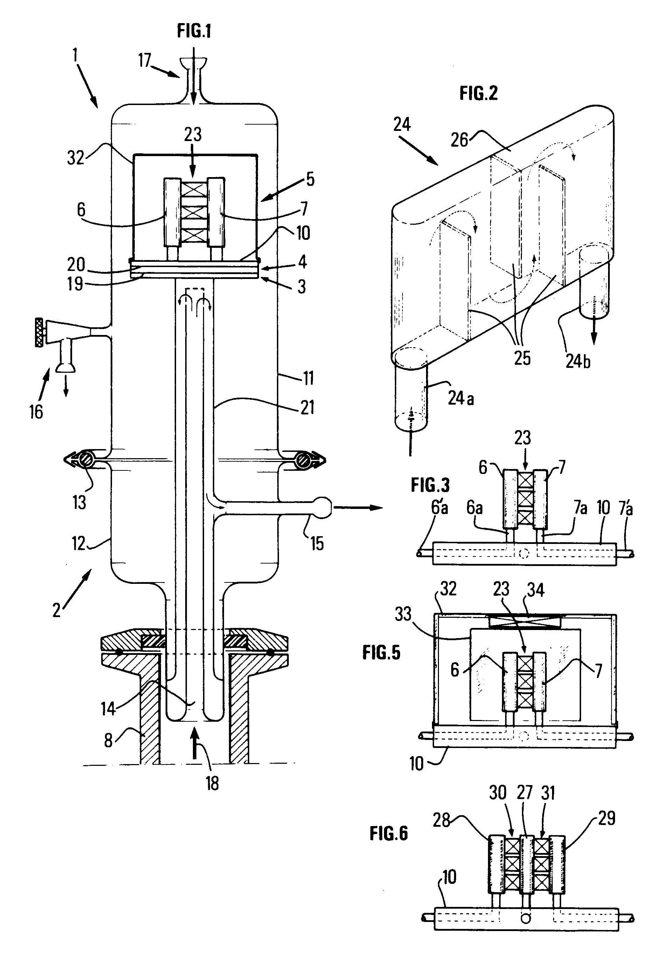

Le calorimètre 1 représenté à la figure 1 comporte une enveloppe 2 contenant une source froide 3, une source chaude 4 et la tête calorimétrique 5, avec ses cellules 6 et 7.

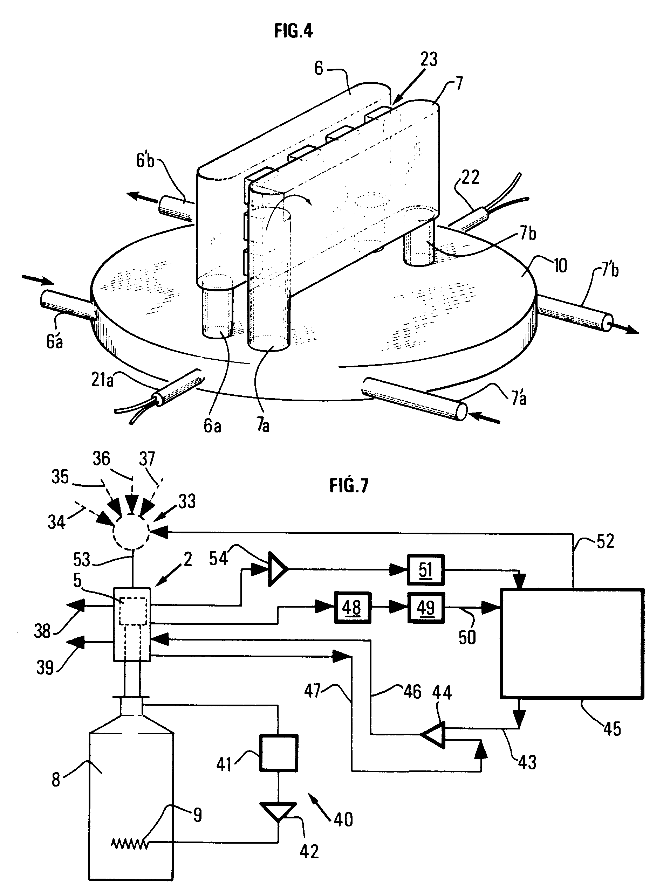

La source de froid 3 est alimentée par de l'azote liquide. Un réservoir 8 (fig. 7) contient une résistance 9 alimentée avec une puissance convenable pour garder une pression constante, indépendante du débit. Une sécurité de niveau d'azote peut compléter ce montage.

L'enveloppe calorimétrique 2 peut être réalisée de manière simple, mais nullement limitative, en verre. Elle peut comporter deux parties supérieures et inférieures 11 et 12 respectivement et donc pouvoir s'ouvrir complètement, son étanchéité étant assurée par un joint torique 13. Elle est munie d'une entrée d'azote froid 14 et de la sortie afférente 15, d'un robinet à vide 16 et d'une ouverture étanche 17 destinée à l'entrée des fils de mesure et de commande, ainsi qu'au passage des tuyaux par lesquels le liquide à analyser s'écoule dans et hors de la tête calorimétrique. Sur la figure, l'ouverture étanche a été représentée sur la partie supérieure 11 de l'enveloppe 2, mais elle pourra avantageusement être placée sur la partie inférieure 12 de cette enveloppe, de manière à dégager plus simplement la tête calorimétrique.

L'azote 18 refroidit une plaque de cuivre 19 soudée au tube de verre 21. Cette plaque 19 qui joue le rôle de la source froide 3 reçoit une résistance chauffante 20 en circuit imprimé qui forme la source chaude 4. Ce circuit imprimé est surmonté d'une deuxième plaque de cuivre 10 qui peut être identique à la première. Cette plaque 10 pourra être qualifiée de four, car elle transmet à la tête calorimétrique la chaleur provenant de la résistance chauffante 20. La plaque 10 (figure 4) comporte deux sondes en platine PT 100, 21A et 22 à quatre fils, dont l'une peut servir à la régulation en température et l'autre à la mesure. Le mode de réalisation décrit correspond à des montages différentiels à cellule couplée.

Une thermopile de mesure 23, par exemple du type 801-3960-01-00-00 commercialisée par la société dite CAMCOOL MODULES reçoit sur chaque face deux cellules étanches 6 et 7, de préférence de mêmes dimensions que la pile. Chaque cellule est reliée au four 10 par deux tubes (tubes 6a et 7a, 7b respectivement) qui assurent ainsi une partie du transfert de chaleur. Enfin ces tubes, après passage dans le four, se prolongent à l'extérieur par des tubes prolongateurs 7'a, 7'b,6'a et 6'b. Ces tubes prolongateurs sortent de l'enveloppe par le passage étanche 17.

Ainsi, on peut changer l'échantillon sans intervenir au niveau des cellules. Leur volume peut être d'environ 300 $\mu$l. La sonde platine de mesure de la température est placée aussi près que possible de la cellule contenant le produit.

La figure 2 représente une cellule référencée 24, cette cellule comporte une canalisation 24a d'entrée du fluide et une cellule 24b de sortie du fluide.

La cellule peut comporter des lamelles 25 formant un système de chicanage qui permet d'avoir un bon balayage de la cellule lorsqu'un nouveau liquide est transféré.

Les canalisations 24a et 24b peuvent se prolonger dans la cellule sensiblement jusqu'au couvercle 26. Dans ce cas, bien entendu, la configuration du système de chicanage sera inverse. Ce mode de réalisation représenté sur la figure 4 pour le tube 7a simplifie la fabrication des cellules et accroît leur rigidité.

Sur la figure 3 on voit en pointillé le système de canalisations 7a, 7'a et 6a, 6'a à travers la plaque 10.

La figure 6 représente un mode de réalisation à trois cellules qui permet de tester deux produits simultanément en microcalorimétrie differentielle à balayage.

La référence 27 désigne la cellule de référence qui est placée entre les cellules de mesure 28 et 29.

Entre la cellule de référence 27 et chacune des cellules de mesure 28 et 29 est placée une thermopile de mesure (thermopiles 30 et 31 respectivement).

Sur la figure 1 la référence 32 désigne le couvercle de la tête calorimétrique. Ce couvercle constitue un écran thermique.

La figure 5 représente un perfectionnement de la tête calorimétrique suivant lequel on utilise un écran intermédiaire 33 relié au couvercle 32 par une thermopile 34. Cette thermopile peut être asservie pour maintenir l'écran 33 à la même température que la deuxième plaque 10. Ainsi les échanges thermiques entre les cellules, 6 et 7, et l'écran intermédiaire 33 sont réduits au minimum.

Le robinet à vide 16 (figure 1) permet d'effectuer le vide dans l'enveloppe 2, ce qui réduit les échanges thermiques entre la tête calorimétrique et les parois de l'enveloppe 2.

Le dispositif selon l'invention est basé sur le principe suivant : lorsqu' on balaye une plage de température dans une enceinte contenant un liquide, on s'aperçoit que la température du liquide suit avec un certain décalage la montée en température de l'enceinte, du moins tant qu'il n'y a pas de changement d'état au sein du liquide. S'il intervient un changement d'état dans le liquide, alors sa température ne suit plus celle de l'enceinte, du moins tant que ce changement n'est pas terminé. C'est la détection du décrochement de la température du liquide par rapport à celle de l'enceinte qui informe sur le changement d'état qui se produit dans le liquide. Cette méthode qui ne nécessite qu'une seule cellule constitue ce qui est appelé la microcalorimétrie à balayage.

Dans le mode de réalisation décrit, on a considéré des dispositifs de microcalorimétrie à balayage différentiel.

Selon cette méthode, il est nécessaire de disposer d'une cellule de mesure, par exemple la cellule 6, et d'une cellule de référence par exemple la cellule 7. Ces deux cellules ayant de préférence les mêmes caractéristiques thermiques. Dans la cellule de mesure on introduit le liquide à tester et dans la cellule de référence un fluide de référence ayant, de préférence, les mêmes caractéristiques thermiques que le fluide à tester, mais ne présentant pas de changement d'état dans la plage de température balayée. On mesure l'écart des températures entre les deux cellules. Celui-ci est faible, si ce n'est nul, du moins tant qu'il n'y a pas de changement d'état dans le fluide à tester.

Losqu'il y a un changement d'état dans le fluide à tester on détecte un écart de température qui permet de déceler le température à laquelle se produit ce changement d'état.

Le dispositif selon la présente invention permet d'automatiser ce processus pour l'évaluation de plusieurs échantillons.

La figure 7 représente un montage permettant cette automatisation.

La référence 33 représente une vanne multivoies permettant d'alimenter la cellule de mesure avec les fluides à tester. Cette vanne peut être commandée par la ligne 52 reliée au calculateur 45. On peut également injecter directement le liquide dans la ligne 53, par exemple par une seringue. Lorsqu'on envoie l'un des fluide à tester par l'une des voies 34, 35, 36 et 37, celui ci chasse l'ancien fluide par la canalisation schématisée en 38.

Il est préférable pour l'obtention de bons résultats que le fluide ne circule pas dans la cellule pendant la période de test. Ceci peut être garanti par l'utilisation de l'organe d'obturation éventuellement commandable des canalisations d'alimentation et/ou de vidange de la cellule. Dans ce cas il sera possible, si nécessaire, d'utiliser un volume tampon afin de compenser les éventuelles variations de volume du produit.

La flèche 39 correspond à la référence 15 de la figure 1 et donc à l'évacuation de l'azote.

La référence 40 désigne un circuit électronique de régulation de la pression à la sortie de la bouteille d'azote liquide 8.

Un système 41 comportant un manomètre à mercure mesure cette pression.

Une photodiode occultée par le niveau du mercure commande par un élément 42 la puissance électrique envoyée dans une résistance 9 immergée dans l'azote liquide. Pour des raisons de sécurité, le chauffage est maximal si la photodiode est éclairée.

La référence 43 désigne la ligne qui fournit à un amplificateur différentiel 44 la température de consigne commandée par le calculateur 45. L'amplificateur 44 fournit au four par la ligne 46 la puissance nécessaire pour obtenir la température de consigne commandée par le calculateur. La régulation de cette température est assurée par la ligne 47 qui est reliée à l'une des sonde de température du four. La mesure de température du four peut être effectuée par un montage à quatre fils avec générateur de courant (1mA) de la sonde Pt 100 de mesure et comporter par exemple un ampli d'instrumentation AD 522 S.

Ainsi la régulation de la température du four peut comporter un préamplificateur, la sonde Pt 100 étant alimentée à intensité constante, un amplificateur d'écart 44 entre la mesure de la température four et la consigne provenant du convertisseur digital-analogique et un amplificateur de puissance.

La mesure de température du calorimètre peut être effectuée de la même manière. La référence 48 désigne le dispositif de mesure de température de la sonde platine. La référence 49 désigne un convertisseur analogique numérique qui peut être du type voltmètre numérique DM-4100 D commercialisé par la société Datel.

Ce convertisseur fournit au calculateur par la ligne 50, une information liée à la température du four.

Un convertisseur 51 du même type pour fournir au calculateur 45 le signal du calorimètre provenant de la thermopile 23 comprise entre les deux cellules 6 et 7.

Avant de parvenir au convertisseur 51, ce signal peut être traité par un amplificateur 54, par exemple du type ampli à Chopper 260 K, commercialisé par la société Analog Device.

Le calculateur peut être du type HP 85 commercialisé par la société Hewlett Packard, complété par des cartes d'entrée-sortie, dites BCD.

Le calculateur 45, sous contrôle de son programme, lit la température et l'état du calorimètre. Il commande le remplissage de la cellule (6) avec le premier échantillon, puis envoie les ordres adéquat au programmateur de température 44 , le test se déroule alors par exemple avec un refroidissement de 0,5 °C/mn et le calculateur effectue l'acquisition des données. Un effet thermique étant détecté, il continue l'acquisition des mesures sur un intervalle de temps ou de température déterminé, puis il remet le calorimètre à la température de départ et effectue le dépouillement de la mesure. Enfin, il commande l'entrée de l'échantillon suivant, et peut procéder à une nouvelle détermination.

L'appareil décrit permet de déterminer le point de trouble des gazoles en se servant de l'effet thermique de cristallisation, sans que l'on fasse appel à un opérateur spécialiste de la calorimétrie. Pour des mesures s'effectuant toujours dans de mêmes conditions notamment de plage de température, on pourra, sans sortir du cadre de la présente invention, utiliser d'autres sources de froid que l'azote liquide. L'avantage important du mode de refroidissement décrit réside dans le possibilité de descendre à de très basses températures, donc d'accéder à des informations complémentaires sur le produit, et en particulier, son analyse fine, par exemple :

a) la température de transition vitreuse (TG) de la matrice hydrocarbonée;

b) la teneur (% poids) de n-paraffines contenues dans le produit étudié;

c) la répartition des paraffines en classe, ou même individuelle, comme les travaux actuels le suggèrent;

Une température de 150 K a été atteinte sans difficulté et il semble possible d'atteindre 120 K.

## Revendications

1. Dispositif de détection et/ou de mesure d'un phénomène thermique intervenant dans un produit présentant un état liquide à une température comprise dans la gamme des températures balayées par ledit dispositif de détection et/ou de mesure, caractérisé en ce qu'il comporte une enceinte (2) comportant une tête calorimétrique (5) contenant au moins une cellule de mesure (6) et au moins une cellule de référence (7), des moyens (10) de transfert de chaleur à ladite cellule (6) de mesure, des moyens de mesure d'une grandeur différentielle relative à la température de ladite cellule de mesure (6), ainsi que des moyens (43-45) de remplissage de ladite cellule (6) de mesure et de la cellule (7) de référence avec ledit liquide et des moyens (16) de vidange de ladite cellule de mesure dudit liquide, ledit dispositif comportant en outre des moyens (48-51; 45) de contrôle de la température régnant dans ladite enceinte (2), lesdits moyens étant adaptés à faire varier la température de l'enceinte (2) sur un intervalle de température prédéterminé.

2. Dispositif selon l'une des revendications précédentes, caractérisé en ce qu'il comporte au moins deux cellules (28, 29) de mesure.

3. Dispositif selon la revendication 2, caractérisé en ce qu'il comporte au moins un fluxmètre tel qu'une thermopile (23) placée entre la cellule de mesure (6) et la cellule de référence (7).

4. Dispositif selon l'une des revendications précédentes, caractérisé en que ladite cellule de mesure (6) est placée sur un socle (10) réalisé en un matériau ayant de bonnes caractéristiques de conductibilité thermique.

**5.** Dispositif selon la revendication 5, caractérisé en ce que ledit socle (10) comporte au moins une portion au moins du circuit d'alimentation de ladite cellule en liquide.

**6.** Dispositif selon l'une des revendications 5 ou 6, caractérisé en ce que ledit socle (10) est en contact avec lesdits moyens de transfert thermique.

## Claims

**1.** A device for detecting and/or measuring a thermal phenomenon occurring in a product having a liquid state at a temperature within the range of temperatures swept by said detecting and/or measuring device, comprising an enclosure (2) with a calorimetric head (5) containing at least one measuring cell (6) and at least one reference cell (7), means (10) for transferring heat to said measuring cell (6), means for measuring a differential magnitude related to the temperature of said measuring cell (6), as well as means (43-45) for filling said measuring cell (6) and reference cell (7) with said liquid and means (16) for emptying said measuring cell of said liquid, said device further comprising means (48-51; 45) for controlling the temperature prevailing in said enclosure (2), said means being adapted for varying the temperature of enclosure (2) within a predetermined temperature range.

**2.** A device as claimed in any one of the previous claims, comprising at least two measuring cells (28, 29).

**3.** A device as claimed in claim 2, comprising at least one flowmeter such as a thermopile (23) located between measuring cell (6) and reference cell (7).

**4.** A device as claimed in any one of the previous claims, wherein said measuring cell (6) is placed on a base (10) made of a material having good thermal conductivity characteristics.

**5.** A device as claimed in claim 4, wherein said base (10) comprises at least a portion of the circuit supplying said cell with liquid.

**6.** A device as claimed in any one of claims 4 or 5, wherein said base (10) is in contact with said heat transfer means.

## Patentansprüche

**1.** Gerät zur Erfassung und/oder Messung eines Wärmephänomens, das in einem Produkt auftritt, das einen flüssigen Zustand bei einer Temperatur im Bereich der durch dieses Erfassung und/oder Meßgerät überstrichenen Temperaturbereichs hat, dadurch gekennzeichnet, daß es einen umschlossenen Raum bzw. eine Kammer (2) umfaßt, die einen kalometrischen Kopf (5), der wenigstens eine Meßzelle (6) und wenigstens eine Bezugszelle (7) enthält, umfaßt, Mitteln (10) zur Überführung von Wärme an diese Meßzelle (6), Mittel zum Messen einer differentiellen Größe, bezogen auf die Temperatur der Meßzelle (6), sowie Mittel (43-45) zum Füllen dieser Meßzelle (6) und der Bezugszelle (7) mit der Flüssigkeit und Mitteln (16) zum Entleeren dieser Meßzelle von dieser Flüssigkeit, wobei das Gerät im übrigen Mittel (48-51; 45) zum Regeln der in diesem umschlossenen Raum (2) herrschenden Temperatur umfaßt, wobei die Mittel so ausgelegt sind, daß sie die Temperatur des umschlossenen Raums bzw. der Kammer (2) über ein vorbestimmtes Temperaturintervall variieren lassen.

**2.** Gerät nach einem der vorhergehenden Ansprüche, dadurch gekennzeichnet, daß es wenigstens zwei Meßzellen (28; 29) umfaßt.

**3.** Gerät nach Anspruch 2, dadurch gekennzeichnet, daß es wenigstens ein Fluxmeter, beispielsweise eine Thermobatterie (23) umfaßt, die zwischen der Meßzelle (6) und der Bezugszelle (7) plaziert ist.

**4.** Gerät nach einem der vorhergehenden Ansprüche, dadurch gekennzeichnet, daß diese Meßzelle (6) auf einem Sockel (10) plaziert ist, der aus einem Material hergestellt ist, welches über gute Eigenschaften der Wärmeleitfähigkeit verfügt.

**5.** Gerät nach Anspruch 4, dadurch gekennzeichnet, daß dieser Sockel (10) wenigstens einen Teil wenigstens des Kreises umfaßt, der diese Zelle mit Flüssigkeit speist.

**6.** Gerät nach einem der Ansprüche 5 oder 6, dadurch gekennzeichnet, daß dieser Sockel (10) in Kontakt mit diesen Wärmeübertragungsmitteln steht.

FIG.1

FIG.2

FIG.3

FIG.5

FIG.6

**FIG.4**

**FIG.7**